(19) Europäisches Patentamt
European Patent Office
Office européen des brevets

(11) Publication number: 0 046 379
B1

(12) # EUROPEAN PATENT SPECIFICATION

(45) Date of publication of patent specification: 16.11.83

(51) Int. Cl.³: C 07 D 213/89,
A 61 K 31/44

(21) Application number: 81303702.5

(22) Date of filing: 14.08.81

(54) **4-Lower hydrocarbyl-3-phenoxypyridine-1-oxides, process for their production, compositions containing them, and their use.**

(30) Priority: 18.08.80 US 179365

(43) Date of publication of application:
24.02.82 Bulletin 82/8

(45) Publication of the grant of the patent:
16.11.83 Bulletin 83/46

(84) Designated Contracting States:
AT BE CH DE FR GB IT LI LU NL SE

(56) References cited:
GB - A - 2 021 113
US - A - 4 187 379

(73) Proprietor: WARNER-LAMBERT COMPANY
201 Tabor Road
Morris Plains New Jersey 07950 (US)

(72) Inventor: Butler, Donald Eugene
1449 Covington Drive
Ann Arbor Michigan 48103 (US)
Inventor: Nordin, Ivan Conrad
340 - 4th Avenue
Holland Michigan 49423 (US)

(74) Representative: Jones, Michael Raymond et al,
Haseltine Lake & Co. 28 Southampton Buildings
Chancery Lane
London WC2A 1AT (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 046 379 B1

Courier Press, Leamington Spa, England.

4-Lower hydrocarbyl-3-phenoxypyridine-1-oxides, process for their production, compositions containing them, and their use

This invention relates to 4-$C_{1-4}$ lower hydrocarbyl-3-phenoxypyridine-1-oxides, a process for producing them, pharmaceutical compositions containing them, and such compounds and compositions in use in the treatment of mammals which may be subject to convulsions, especially epileptic seizures.

According to one aspect of the present invention, there is provided a 4-$C_{1-4}$ lower hydrocarbyl-3-phenoxypyridine-1-oxide, or its hydrate.

In accordance with another aspect of the present invention, 4-$C_{1-4}$ lower hydrocarbyl-3-phenoxypyridine-1-oxides having the formula:

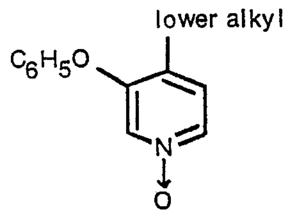

may be produced by reacting 4-$C_{1-4}$ lower hydrocarbyl-3-phenoxypyridines with an oxidizing agent of the type generally employed to convert amines to N-oxides, such as 3—30 percent hydrogen peroxide in water; 5—40 percent peracetic acid in acetic acid; perbenzoic acid; perfluoroacetic acid; perphthalic acid; or *m*-chloroperbenzoic acid; with the preferred oxidizing agent being 40 percent peracetic acid. Although equimolar amounts of the two reactants may be employed, preferably an excess of oxidizing agent is used. While the amount in excess is not critical, one may use as much as a two fold excess or even more. The solvent system may be any generally used in oxidation reactions of this type with the preferred being glacial acetic acid; mixtures of water in acetic acid; and halogenated hydrocarbons such as dichloromethane, chloroform or tetrachloroethane. The most preferred is glacial acetic acid. The reaction is conveniently carried out at a temperature in the range from 0°C to 100°C for from one to twenty-four hours, preferably 55°C to 60°C for approximately sixteen hours. The product may be isolated by distillation.

The term "lower hydrocarbyl" as used herein is intended to means a hydrocarbon moiety having from one to four carbon atoms such as methyl, ethyl, propyl, iso-propyl, iso-propyl, *n*-butyl, *s*-butyl, *t*-butyl; cyclopropyl, cyclobutyl, propenyl, allyl and 1-butenyl. The preferred compound is that wherein the lower hydrocarbyl is a "lower alkyl", especially methyl.

The compounds of the present invention include solvates such as the hydrate, as well as stereoisomers or regioisomers and mixtures thereof depending upon the structure of the lower hydrocarbyl group.

In the following description reference will be made to the lower alkyl compounds and yet it is to be appreciated that the invention extends to other lower hydrocarbyl compounds.

4-Lower alkyl-3-phenoxypyridine-1-oxides may be administered for the purpose of treating convulsions or preventing the onset of convulsions in mammals, such as rodents, dogs and cats. The specific profile of this class of compounds indicates its use would be in treating conditions where antiepileptic agents such as phenytoin and carbamazepine and to a lesser extent mephenytoin are traditionally employed.

The utility of the aforementioned class of compounds as anticonvulsants is determined by generating a compound profile using three tests. The tests employed are described in Epilepsia *19* (1978) 409—428 which is incorporated by reference.

The tests were performed by the National Institute of Neurological and Communicative Disorders and Stroke and used male Carworth Farms No. 1 mice. The compounds are employed at at least 3 dose levels (30, 100, 300 mg/kg) in the following three tests.

Maximal Electroshock Seizure Test—(MES)

Maximal electroshock seizures are elicited with a 60 cycle alternating current of 50 mA intensity (5—7 times that necessary to elicit minimal electroshock seizures) delivered for 0.2 sec via corneal electrodes. A drop of 0.9% saline is instilled in the eye prior to application of the electrodes in order to prevent the death of the animal. Abolition of the hind limb tonic extension component of the seizure is defined as protection, and results are expressed as:

number of animals protected/number of animals tested.

Subcutaneous Pentylenetetrazol (Metrazol) Seizure Threshold Test — (sc Met).

85 mg/kg of pentylenetetrazol (which produces seizures in more than 95% of mice) is administered as a 0.5% solution subcutaneously in the posterior midline. The animal is observed for 30

minutes. Failure to observe even a threshold seizure (a single episode of clonic spasms of at least 5 seconds duration) is defined as protection, and the results are expressed as:
number of animals protected/number of animals tested.

Toxicity Test — (Tox)

The Rotorod test is used to evaluate neurotoxicity. The animal is placed on a 1 inch diameter knurled plastic rod rotating at 6 rpm. Normal mice can remain on a rod rotating at this speed indefinitely. Neurologic toxicity as defined as the failure of the animal to remain on the rod for 1 minute, and is expressed as:

number of animals exhibiting toxicity/number of animals tested.

Profile of Anticonvulsant Activity on
*Orally* Administered 4-Methyl-3-Phenoxypyridine-1-Oxide
Some Prototype Antiepileptics in Mice

| Substance | Time of Test (hrs) | | ROTOROD TD50 (mg/kg) | | MES–ED50 (mg/kg) | | sc Met–ED50 (mg/kg) | |
|---|---|---|---|---|---|---|---|---|
| | Mice | Rats | Mice | Rats | Mice | Rats | Mice | Rats |
| 4-Methyl-3-Phenoxy-Pyridine-1-Oxide | 1/2, 1/2,– | | 296.18<br>(250.60–344.01)<br>[8.32] | 76.07<br>(58.13–94.79)<br>[5.16] | 3.47<br>85.29<br>(68.68–102.95)<br>[5.26] | 6.80<br>11.19<br>(8.77–13.80)<br>[5.89] | Potentiates | Potentiates |
| Phenytoin | 2,2,– | 1/2,4 | 86.71<br>(80.39–96.09)<br>[13.01] | >3000 | 9.59<br>9.04<br>(7.39–10.62)<br>[6 .28] | >100<br>29.82<br>(21.92–38.91)<br>[2.82] | Ineffective | Ineffective |
| Mepheny-toin | 1 1/2, 1 1/2 | 2,2 | 97.0*<br>(85.0–110.6) | 143*<br>(106–193) | 2.62<br>37.0<br>(24.8–55.1) | 14.30<br>10<br>(8.7–11.6) | 1.83<br>53.0<br>(35.8–78.5) | 1.30<br>105<br>(72–154) |
| Carbama-zepine | 1/2, 1/2 | 2,1 | 217.21<br>(131.49–270.11)<br>[3.47] | 813.06<br>(488.76–1233.87)<br>[6.07] | 14.06<br>15.44<br>(12.44–17.31)<br>[9.07] | 95.65<br>8.50<br>(3.39–10.53)<br>[4.50] | 4.52<br>48.07<br>(40.75–57.35)<br>[5.50] | Variable |

*Data from Swinyard, Brown, and Goodman. J. Pharmacol. & Exper. Therap. *106*, 319–330, 1952,

**Data from Swinyard, Orr, Jolley, and Goodman. Fed. Proc. *9*, 319, 1950.

[ ] Slope, regression line.

( ) 95% Confidence intervals.

Data in small square is a protective index. The protective index is the rotorod toxicity dose divided by the minimal effective anticonvulsant dose.

Profile of Anticonvulsant Activity of
*Intraperitoneally* Administered 4-Methyl-3-Phenoxypyridine-1-Oxide
Some Prototype Antiepileptics in Mice

| Substance | Time of Test (hrs) | TD50 (mg/kg) | ED50 (mg/kg) and PI | | | | |
|---|---|---|---|---|---|---|---|
| | | | MES | sc Met | Bicuculline | Picrotoxin | Strychnine |
| 4-Methyl-3-Phenoxy-Pyridine-1-Oxide | 1/2, 1/2 | 76.25 (71.34–80.89) [24.50] | 3.28<br>23.22 (20.53–26.61) [11.23] | Potentiates | Potentiates | Potentiates | Ineffective |
| Phenytoin | 2,2 | 65.46 (52.49–72.11) [15.23] | 6.89<br>9.50 (8.13–10.44) [13.66] | Potentiates | Potentiates | Potentiates | Max. Prot. 50% at 55–100 mg/kg |
| Mephenytoin | 1/4, 1/2 | 153.82 (132.86–178.73) [9.22] | 2.54<br>60.50 (49.45–70.25) [8.01] | 5.05<br>30.45 (19.67–39.47) [4.76] | 1.24<br>124.14 (84.10–188.49) [1.99] | 1.52<br>100.96 (79.34–122.92) [7.43] | Max. Prot. 50% at 70–150 mg/kg |
| Carbamazepine | 1/4, 1/4 | 71.56 (45.91–134.79) [4.77] | 8.12<br>8.81 (5.45–14.09) [3.62] | Potentiates | Max. Prot. 62.5% at 50–130 mg/kg. | 1.92<br>37.20 (25.32–59.69) [3.86] | 0.91<br>78.83 (39.39–132.03) [2.85] |

( ) Confidence Levels, 95%.

[ ] Slope, regression line.

Data in small square is a Protective Index. The Protective Index is the rotorod toxicity dose divided by the minimal effective anticonvulsant dose.

4-Lower alkyl-3-phenoxypyridine-1-oxides are useful anticonvulsants in mammals such as dogs, cats, horses and sheep, when administered in amounts ranging from 0.014 mg to 21.4 mg per kg of body weight per day. A preferred dosage regimen for optimum results would be from 0.36 mg to 10.7 mg per kg of body weight per day, and such dosage units are employed that a total of from 1 mg to 1500 mg of active ingredient for a subject of about 70 kg body weight are administered in a 24 hour period, preferably 25 mg of 750 mg/day.

The compounds of the present invention in the described dosages are intended to be administered orally; however, other routes such as rectally, intraperitoneally, intramuscularly or intravenously may be employed. Of the routes other than oral, the most important is the intravenous route.

The active compounds of the present invention may be orally administered with, for example, an inert diluent or an assimilable edible carrier, or they may be enclosed in hard or soft gelatin capsules. In addition, they may be compressed into tablets, or may be incorporated directly with the food of the diet. For oral therapeutic administration, the active compounds of this invention may be incorporated with excipients and used in the form of tablets, troches, or capsules. Other less frequently used solid formulations include wafers, chewing gum, and the like. Liquid formulations may include solutions or suspensions (e.g. elixirs or syrups). Such compositions and preparations should contain at least 0.1% of active compound. The percentage in the compositions and preparations may, of course, be varied and may conveniently be between 5% to 75% or more of the weight of the unit. The amount of active compound in such therapeutically useful compositions or preparations is such that a suitable dosage will be obtained. Preferred compositions or preparations according to the present invention are prepared so that an oral dosage unit form contains from 1.0 to 50 milligrams of active compound.

The tablets, troches, pills, capsules and the like may also contain the following: a binder such as gum tragacanth, acacia, corn starch or gelatin; an excipient such as dicalcium phosphate; a disintegrating agent such as corn starch, potato starch or alginic acid; and a lubricant such as magnesium stearate. Also, a sweetening agent such as sucrose, lactose or saccharin may be added or a flavouring agent such as peppermint, oil of wintergreen, or cherry flavouring. When the dosage unit form is a capsule, it may contain in addition to materials of the above type of liquid carrier, such as a fatty oil or a solid carrier, such as lactose. Various other materials may be present as coatings or to modify otherwise the physical form of the dosage unit; for instance, tablets, pills or capsules may be coated with shellac, sugar or both. A syrup or elixir may contain the active compounds, sucrose as a sweetening agent, methyl and propyl parabens as preservatives, a dye and a flavouring such as cherry or orange flavour. Of course, any material used in preparing any dosage unit form should be pharmaceutically pure and substantially non-toxic in the amounts employed.

When used in the form of a suppository, the compounds of the present invention should be dispersed in a carrier, such as glycerine or cacao butter.

Parenteral preparations such as those of the intravenous or intramuscular type, may be in the form of sterilized aqueous solutions containing buffering agents, preservatives, salts to control isotonisity, or lyphilized materials which may be either the pure sterilized compound or a mixture containing additives, such as buffering agents (phosphate buffers), salts (sodium chloride), or preservatives (benzalkonium chloride). The pure sterile compound can be dissolved in standard injectable solutions, such as Ringer's Solution, while the mixtures can be dissolved in water for injection.

The present invention will be described in greater detail in conjunction with the following specific examples.

Example 1

A solution of 25 g of 4-methyl-3-phenoxypyridine, (J. Med. Chem., 18, 1 (1975)) in 90 ml of glacial acetic acid was treated with 30 ml of 40% peracetic acid in acetic acid and the mixture was stirred and heated at 60°C for 12 hours. Two further 10 ml portions of 40% peracetic acid were added and the mixture heated for 4 hours at 60°C. Then 100 ml of isopropanol were added and the mixture was heated at 90°C for 4 hours. The resulting mixture was stripped at reduced pressure and dissolved in 500 ml of dichloromethane. The organic layer was washed with an excess of 25% sodium hydroxide solution, dried over anhydrous magnesium sulphate, filtered, concentrated, and distilled to yield 4-methyl-3-phenoxypyridine-1-oxide. B.P. 150—152°C/0.3 mm Hg (39.99 Pa)

Example 2

| Ingredient: | Quantity: |
|---|---|
| 4-methyl-3-phenoxypyridine-1-oxide | 150 g |
| lactose | 1124 g |
| corn starch | 39 g |
| hydroxypropyl cellulose | 30 g |
| magnesium stearate | 7 g |
| ethanol-water 50:50 | qs |

The 4-methyl-3-phenoxypyridine-1-oxide, and hydroxypropyl cellulose were blended and granulated with 50:50 ethanol-water. The wet granulation was screened, dried, and rescreened. The resulting dried granulation was blended with magnesium stearate and the corn starch, and the mixture was compressed into 225 mg tablets using 11/32 inch (8.7 mm) standard concave punches. There were produced approximately 6000 tablets each of which contained 25 mg of 4-methyl-3-phenoxypyridine-1-oxide.

Example 3

| Ingredient: | Quantity: |
|---|---|
| 4-methyl-3-phenoxypyridine-1-oxide | 15 g |
| lactose | 1249 g |
| corn starch | 39 g |
| hydroxypropyl cellulose | 30 g |
| magnesium stearate | 7 g |
| ethanol-water 50:50 | qs |

The 4-methyl-3-phenoxypyridine-1-oxide, lactose and hydroxypropyl cellulose were blended and granulated with 50:50 ethanol-water. The wet granulation was screened, dried, and rescreened. The resulting dried granulation was blended with magnesium stearate and the corn starch, and the mixture was compressed into 225 mg tablets using 11/32 inch (8.7 mm) standard concave punches. There were produced approximately 6000 tablets each of which contained 2.5 mg of 4-methyl-3-phenoxypyridine-1-oxide.

Example 4

| Ingredient: | Quantity: |
|---|---|
| 4-methyl-3-phenoxypyridine-1-oxide | 6 g |
| lactose | 1268 g |
| corn starch | 39 g |
| hydroxypropyl cellulose | 30 g |
| magnesium stearate | 7 g |
| ethanol-water 50:50 | qs |

The 4-methyl-3-phenoxypyridine-1-oxide, lactose and hydroxypropyl cellulose were blended and granulated with 50:50 ethanol-water. The wet granulation was screened, dried, and rescreened. The resulting dried granulation was blended with magnesium stearate and the corn starch, and the mixture was compressed into 225 mg tablets using 11/32 inch (8.7 mm) standard concave punches. There were produced approximately 6000 tablets each of which contained 1.0 mg of 4-methyl-3-phenoxypyridine-1-oxide.

Example 5

| Ingredient: | Quantity: |
|---|---|
| 4-methyl-3-phenoxypyridine-1-oxide | 300 g |
| lactose | 974 g |
| corn starch | 39 g |
| hydroxypropyl cellulose | 30 g |
| magnesium stearate | 7 g |
| ethanol-water 50:50 | qs |

The 4-methyl-3-phenoxypyridine-1-oxide, lactose and hydroxypropyl cellulose were blended and granulated with 50:50 ethanol-water. The wet granulation was screened, dried, and rescreened. The resulting dried granulation was blended with magnesium stearate and the corn starch, and the mixture was compressed into 225 tablets using 11/32 inch (8.7 mm) standard concave punches. There were produced approximately 6000 tablets each of whcih contained 50 mg of 4-methyl-3-phenoxy-pyridine-1-oxide.

Example 6

| Ingredient: | Quantity: |
|---|---|
| 4-methyl-3-phenoxypyridine-1-oxide | 250 g |
| lactose | 1723 g |
| magnesium stearate | 27 g |

The mixture was blended and introduced into No. 4 hard gelatin capsules, filling each capsule with 200 mg of the powder mixture. There were produced approximately 10,000 capsules each of which contained 25 mg of 4-methyl-3-phenoxypyridine-1-oxide.

Example 7

| Ingredient: | Quantity: |
|---|---|
| 4-methyl-3-phenoxypyridine-1-oxide | 25 g |
| lactose | 1948 g |
| magnesium stearate | 27 g |

The mixture was blended and introduced into No. 4 hard gelatin capsules, filling each capsule with 200 mg of the powder mixture. There were produced approximately 10,000 capsules each of which contained 2.5 mg of 4-methyl-3-phenoxypyridine-1-oxide.

Example 8

| Ingredient: | Quantity: |
|---|---|
| 4-methyl-3-phenoxypyridine-1-oxide | 250 g |
| lactose | 1963 g |
| magnesium stearate | 27 g |

The mixture was blended and introduced into No. 4 hard gelatin capsules, filling each capsule with 224 mg of the powder mixture. There were produced approximately 10,000 capsules each of which contained 25 mg of 4-methyl-3-phenoxypyridine-1-oxide.

Example 9

| Ingredient: | Quantity: |
| --- | --- |
| 4-methyl-3-phenoxypyridine-1-oxide | 500 g |
| lactose | 1473 g |
| magnesium stearate | 27 g |

The mixture was blended and introduced into No. 4 hard gelatin capsules, filling each capsule with 200 mg of the powder mixture. There were produced 10,000 capsules each of which contained 50 mg of 4-methyl-3-phenoxypyridine-1-oxide.

Example 10

| Ingredient: | Quantity: |
| --- | --- |
| 4-methyl-3-phenoxypyridine-1-oxide | 1.0 g |
| phemerol chloride recrystallized | 0.1 g |
| water for injection USP | qs ad 1.0 ml |

The 4-methyl-3-phenoxypyridine-1-oxide was mixed with about two-thirds of the required volume of water for injection USP followed by the addition of sufficient water for injection to reach the desired volume. After mixing, the solution was sterilized by membrane filtration (a 0.22 micron Millipore filter membrane represented a suitable filter). The desired quantity of the above prepared solution was introduced into appropriate size multiple dose vials suitable for injection preparations and stoppered with gum rubber or suitable rubber closures and sealed with aluminium ferrules. The preparation may also be filled into suitable size single dose glass ampoules and sealed.

Using the above procedure, solutions containing 1.0, 2.5, 5.0, or 10.0 mg/ml of 4-methyl-3-phenoxypyridine-1-oxide may be prepared.

**Claims for the Contracting States: BE CH DE FR GB IT LI LU NL SE**

1. A $C_{1-4}$ lower hydrocarbyl-3-phenoxypyridine-1-oxide, or its hydrate.
2. A compound according to Claim 1, wherein the lower hydrocarbyl radical is methyl, ethyl, propyl, isopropyl, *n*-butyl, *s*-butyl, *t*-butyl, cyclopropyl, cyclobutyl, propenyl, allyl or 1-butenyl.
3. 4-methyl-3-phenoxypyridine-1-oxide, or its hydrate.
4. A pharmaceutical composition which comprises a compound according to Claim 1, 2 or 3, and a pharmaceutical carrier therefor.
5. A pharmaceutical composition according to Claim 4, which is in unit dosage form.
6. A pharmaceutical composition according to Claim 5, which contains from 0.5 mg to 100 mg of 4-lower hydrocarbyl-3-phenoxypyridine-1-oxide per unit dose.
7. A pharmaceutical composition according to Claim 6, which contains from 1.0 mg to 50 mg of 4-methyl-3-phenoxypyridine-1-oxide per unit dose.
8. A process of producing a 4-lower hydrocarbyl $C_{1-4}$-3-phenoxypyridine-1-oxide, which comprises reacting a 4-$C_{1-4}$ lower hydrocarbyl-3-phenoxypyridine with an oxidizing agent.
9. A process according to Claim 8, wherein the oxidizing agent is peracetic acid.
10. A process according to Claim 9, wherein lower hydrocarbyl is methyl.

**Claims for the Contracting State: AT**

1. A process for producing a 4-$C_{1-4}$ lower hydrocarbyl-3-phenoxy-pyridine-1-oxide, which comprises reacting a 4-$C_{1-4}$ lower hydrocarbyl-3-phenoxypyridine with an oxidizing agent.
2. A process according to Claim 1, wherein the oxidizing agent is hydrogen peroxide in water, peracetic acid, perphthalic acid or *m*-chloroperbenzoic acid.
3. A process according to Claim 1, wherein the oxidizing agent is peracetic acid.
4. A process according to Claim 1, 2 or 3, wherein the oxidizing agent is employed in a molar excess with respect to the 4-lower hydrocarbyl-3-phenoxy-pyridine.
5. A process according to any preceding claim, wherein the reaction is effected in glacial acetic acid as solvent.
6. A process according to any preceding claim, wherein the reaction is effected as from 55°C to 60°C for approximately 16 hours.
7. A process according to any preceding claim, wherein the lower hydrocarbyl radical of the starting material is methyl, ethyl, propyl, isopropyl, *n*-butyl, *s*-butyl, *t*-butyl, cyclopropyl, cyclobutyl, propenyl, allyl or 1-butenyl.
8. A process for producing a pharmaceutical composition, which comprises incorporating in a

pharmaceutically-acceptable carrier a 4-$C_{1-4}$ lower hydrocarbyl-3-phenoxy-pyridine-1-oxide or its hydrate.

9. A process according to Claim 8, wherein the active ingredient is 4-methyl-3-phenoxy-pyridine-1-oxide.

10. A process according to Claim 8 or 9, wherein the process results in a composition in unit dosage form, with each dose containing from 0.5 to 100 mg of 4-$C_{1-4}$ lower hydrocarbyl-3-phenoxy-pyridine-1-oxide.

**Revendications pour les Etats contractants: BE CH DE FR GB IT LI LU NL SE**

1. $C_{1-4}$ hydrocarbyl inférieur-3-phénoxypyridine-1-oxyde ou son hydrate.

2. Composé suivant la revendication 1, dans lequel le radical hydrocarbyle inférieur est un méthyle, éthyle, propyle, isopropyle, n-butyle, s-butyle, t-butyle, cyclopropyle, cyclobutyle, propényle, allyle ou 1-butényle.

3. 4-méthyl-3-phénoxypyridine-1-oxyde ou son hydrate.

4. Composition pharmaceutique contenant un composé suivant la revendication 1, 2 ou 3, et un véhicule pharmaceutique pour celui-ci.

5. Composition pharmaceutique suivant la revendication 4, se trouvant sous la forme de dosage unitaire.

6. Composition pharmaceutique suivant la revendication 5, contenant de 0,5 mg à 100 mg de 4-hydrocarbyl inférieur-3-phénoxypyridine-1-oxyde par dose unitaire.

7. Composition pharmaceutique suivant la revendication 6, contenant de 1,0 mg à 50 mg de 4-méthyl-3-phénoxypyridine-1-oxyde par dose unitaire.

8. Procédé de préparation d'un 4-hydrocarbyl inférieur en $C_{1-4}$-3-phénoxypyridine-1-oxyde consistant à faire réagir un 4-$C_{1-4}$ hydrocarbyl inférieur-3-phénoxypyridine avec un agent d'oxydation.

9. Procédé suivant la revendication 8, dans lequel l'agent d'oxydation est l'acide péracétique.

10. Procédé suivant la revendication 8, dans lequel l'hydrocarbyle inférieur est le méthyle.

**Revendications pour l'Etat contractant: AT**

1. Procédé de préparation d'un 4-$C_{1-4}$ hydrocarbyl inférieur-3-phénoxypyridine-1-oxyde, consistant à faire réagir une 4-$C_{1-4}$ hydrocarbyl inférieur-3-phénoxypyridine avec un agent d'oxydation.

2. Procédé suivant la revendication 1, dans lequel l'agent d'oxydation est le péroxyde d'hydrogène dans l'eau, l'acide péracétique, l'acide perphtalique ou l'acide m-chloroperbenzoïque.

3. Procédé suivant la revendication 1, dans lequel l'agent d'oxydation est l'acide péracétique.

4. Procédé suivant la revendication 1, 2 ou 3, dans lequel l'agent d'oxydation est employé en excès molaire par rapport à la 4-hydrocarbyl inférieur-3-phénoxypyridine.

5. Procédé suivant l'une quelconque des revendications précédentes, dans lequel la réaction est effectuée dans l'acide acétique glacial comme solvant.

6. Procédé suivant l'une quelconque des revendications précédentes, dans laquelle la réaction est effectuée à une température de 55°C à 60°C pendant environ 16 heures.

7. Procédé suivant l'une quelconque des revendications précédentes, dans lequel le radical hydrocarbyle inférieur du matériau de départ est un méthyle, éthyle, propyle, isopropyle, n-butyl, s-butyle, t-butyle, cyclopropyle, cyclobutyle, propényle, allyle ou 1-butényle.

8. Procédé de préparation d'une composition pharmaceutique consistant à incorporer dans un véhicule pharmaceutiquement acceptable un 4-$C_{1-4}$ hydrocarbyl inférieur-3-phénoxypyridine-1-oxyde ou son hydrate.

9. Procédé suivant la revendication 8, dans lequel l'ingrédient actif est le 4-méthyl-3-phénoxypyridine-1-oxyde.

10. Procédé suivant la revendication 8 ou 9, dans lequel le procédé a comme résultat une composition sous forme de dosage unitaire, chaque dose contenant de 0,5 à 100 mg de 4-$C_{1-4}$ hydrocarbyl inférieur-3-phénoxypyridine-1-oxyde.

**Patentansprüche für die Vertragsstaaten: BE CH DE FR GB IT LI LU NL SE**

1. $C_{1-4}$-Niedrighydrocarbyl-3-phenoxypyridin-1-oxid oder sein Hydrat.

2. Verbindung nach Anspruch 1, dadurch gekennzeichnet, daß das Niedrighydrocarbylradikal Methyl, Äthyl, Propyl, Isopropyl, n-Butyl, s-Butyl, t-Butyl, Cyclopropyl, Cyclobutyl, Propenyl, Allyl oder 1-Butenyl ist.

3. 4-Methyl-3-phenoxypyridin-1-oxid oder sein Hydrat.

4. Pharmazeutische Zusammensetzung, dadurch gekennzeichnet, daß sie eine Verbindung nach Anspruch 1, 2 oder 3 und einen pharmazeutischen Träger dafür enthält.

5. Pharmazeutische Zusammensetzung nach Anspruch 4 in Dosiseinheitsform.

6. Pharmazeutische Zusammensetzung nach Anspruch 5, welche von 0,5 mg bis 100 mg 4-Niedrighydrocarbyl-3-phenoxypyridin-1-oxid pro Dosiseinheit enthält.

7. Pharmazeutische Zusammensetzung nach Anspruch 6, welche von 1,0 mg bis 50 mg 4-Methyl-3-phenoxypyridin-1-oxid pro Dosiseinheit enthält.

8. Verfahren zur Herstellung eines 4-Niedrighydrocarbyl-$C_{1-4}$-3-phenoxypyridin-1-oxids, dadurch gekennzeichnet, daß ein 4-$C_{1-4}$-Niedrighydrocarbyl-3-phenoxypyridin mit einem Oxidationsmittel umgesetzt wird.

9. Verfahren nach Anspruch 8, dadurch gekennzeichnet, daß das Oxidationsmittel Peressigsäure ist.

10. Verfahren nach Anspruch 9, dadurch gekennzeichnet daß Niedrighydrocarbyl Methyl bedeutet.

**Patentansprüche für den Vertragsstaat: AT**

1. Verfahren zur Herstellung eines 4-$C_{1-4}$-Niedrighydrocarbyl-3-phenoxy-pyridin-1-oxids, dadurch gekennzeichnet, daß ein 4-$C_{1-4}$-Niedrighydrocarbyl-3-phenoxypyridin mit einem Oxidationsmittel umgesetzt wird.

2. Verfahren nach Anspruch 1, dadurch gekennzeichnet, daß das Oxidationsmittel Wasserstoffperoxid in Wasser, Peressigsäure, Perphthalsäure oder m-Chlorperbenzoesäure ist.

3. Verfahren nach Anspruch 1, dadurch gekennzeichnet, daß das Oxidationsmittel Peressigsäure ist.

4. Verfahren nach Anspruch 1, 2 oder 3, dadurch gekennzeichnet, daß das Oxidationsmittel in molarem Überschuß in bezug auf das 4-Niedrighydrocarbyl-3-phenoxy-pyridin verwendet wird.

5. Verfahren nach jedem der vorhergehenden Ansprüche, dadurch gekennzeichnet, daß die Umsetzung in Eisessig als Lösungsmittel durchgeführt wird.

6. Verfahren nach jedem der vorhergehenden Ansprüche, dadurch gekennzeichnet, daß die Umsetzung bei 55°C bis 60°C während annähernd 16 Stunden durchgeführt wird.

7. Verfahren nach jedem der vorhergehenden Ansprüche, dadurch gekennzeichnet, daß das Niedrighydrocarbylradikal des Ausgansmaterials Methyl, Äthyl, Propyl, Isopropyl, n-Butyl, s-Butyl, t-Butyl, Cyclopropyl, Cyclobutyl, Propenyl, Allyl oder 1-Butenyl ist.

8. Verfahren zur Herstellung einer pharmazeutischen Zusammensetzung, dadurch gekennzeichnet, daß in einen pharmazeutisch akzeptablen Träger ein 4-$C_{1-4}$-Niedrighydrocarbyl-3-phenoxy-pyridin-1-oxid oder sein Hydrat inkorporiert wird.

9. Verfahren nach Anspruch 8, dadurch gekennzeichnet, daß das aktive Ingrediens 4-Methyl-3-phenoxy-pyridin-1-oxid ist.

10. Verfahren nach Anspruch 8 oder 9, dadurch gekennzeichnet, daß das Verfahren zu einer Zusammensetzung in Dosiseinheitsform führt, wobei jede Dosis von 0,5 bis 100 mg 4-$C_{1-4}$-Niedrighydrocarbyl-3-phenoxy-pyridin-1-oxid enthält.